# EUROPEAN PATENT APPLICATION

(11) **EP 4 438 052 A1**
(43) Date of publication of application: **02.10.2024**
(21) Application number: 23165695.0
(22) Date of filing: 30.03.2023
(51) Int. Cl.: A61K 36/81, A61P 31/10, A01N 65/40

(54) **PLANT EXTRACT FOR USE IN TREATMENT OF A FUNGAL INFECTION**

(71) Applicant: FytagoLife B.V., 3232 HE Brielle (NL)
(72) Inventor: KORTHOUT, Henricus Adriaan Anna Jacobus, 2333 BE Leiden (NL); KIM, Hye Kyong, 2311 EZ Leiden (NL); DE GRAAF, Esmée France, 2311 EZ Leiden (NL); VAN DUIJN, Albert, 2333 BE Leiden (NL); VAN HORSSEN, Frederik Pius, 2333 BE Leiden (NL); VAN DEN HONDEL, Cornelis Antonius Maria Jacobus Johannes, 2333 BE Leiden (NL)
(74) Representative: V.O.

(57) **Abstract**

The invention relates to a plant extract from a food crop residue and sebacic acid or a salt thereof for use in a method of treatment of a fungal infection, said food crop belonging to the family of Solanaceae. The invention further relates to a composition comprising a plant extract from a food crop residue belonging to the family of Solanaceae or sebacic acid and a suitable carrier.

## Description

The invention relates to a plant extract, sebacic acid or a composition comprising a plant extract or sebacic acid for use in a method of treatment of fungal infections and a use of a plant extract, sebacic acid or a composition comprising the plant extract or sebacic acid as disinfectant and an object treated with a plant extract or sebacic acid. The invention further relates to a composition comprising a plant extract or sebacic acid and a suitable carrier.

Fungal infections throughout the world still appear to be increasing. A broad spectrum of current fungal infections are caused by invasive and non-invasive fungi and their medical impact may vary from psychological problems to death. Onychomycosis or "fungal nail" is a typical example of a fungal infection with a high psychological impact, affecting approximately 5.5% of the world's population (Lipner and Scher, 2019). It is caused by dermatophytes, non-dermatophytic molds, and yeast. Dermatophytes are the primary causative agent with *Trichophyton nientagrophytes* and *Trichophyton rubrum* as the main causes of onychomycoses, responsible for 20% and 71% of all infections respectively (Faergemann and Baran, 2003). The most prevalent form of onychomycosis is distal and lateral subungual onychomycosis, in which the fungi invade through a fissure in the skin at the lateral or distal undersurface of the nail. Subsequently, the fungi may spread subungual where hyperkeratosis can lead to the separation of the nail from the nailbed, called onycholysis (Brown et al., 2018; Westerberg and Voyack, 2013). Infection by these types of fungi also frequently causes discoloration, brittleness, thickened nails (onychogryphosis), splitting, and receded lunula's (Shirwaikar et al., 2008; Westerberg and Voyack, 2013). It can cause unsightly nuisance, discomfort, and pain in patients.

There are currently several ways to treat onychomycosis, including devices (like photodynamic therapy or laser treatments), oral therapies, topical therapies, or even nail avulsions (Baswan et al., 2017; Brown et al., 2018). Topical treatments are frequently used. These therapies make localized delivery of the drug possible and eliminate potential severe side effects for patients. However, current marketed topical products have low clearance rates and are inadequate (Christenson et al., 2018; Del Rosso, 2014; Gupta and Stec, 2019).

Further, many topical therapies lack the ability to penetrate through the nail and, subsequently, kill the fungi. Consequently, leaving most available topical treatments ineffective (Elkeeb et al., 2017).

Accordingly, there is a need for an alternative therapy for the treatment of fungal infections, and in particular fungal infections caused by *Trichophyton,* such as onychomycosis.

The inventors surprisingly realized that from food crop residue of the family of Solanaceae, a plant extract may be prepared that exhibits antifungal activity. In particular, said extract was found to be active against *Trichophyton* species such as *T. mentagrophytes* and *T. rubrum* that are underlying to onychomycosis.

The inventors further surprisingly realized that the efficacy of the plant extract may be further enhanced by administering the plant extract in a composition with one or more Natural Deep Eutectic Solvents (NADES).

Without wishing to be bound by any theory, it is believed that such a composition has an improved ability to penetrate through keratinized structures, such as nails, to effectively reach the fungi that reside beneath these structures, as is the case in onychomycosis.

Accordingly, the invention relates to a plant extract from a food crop residue from the family *Solanaceae* for use in a method of treatment of a fungal infection, preferably a fungal infection caused by Trichophyton, more preferably T. *mentagrophytis* and/or *T. rubrum,* in particular for use in a method of treatment of onychomycosis.

### Figures

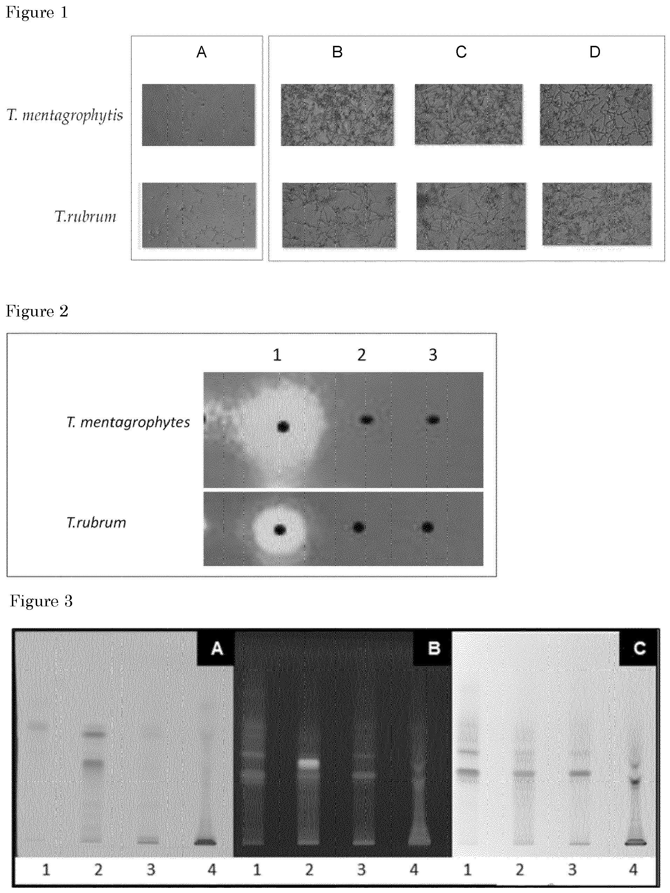

Figure 1: Microtiter plate assay to test the antifungal activity of a tomato extract against *T. mentagrophytes* and *T. rubrum.* A: Tomato extract; B: H₂O (control); C: 50% ethanol (control); D: DMSO (control).
Figure 2: Spot inhibition assay to test the antifungal activity of a tomato extract dissolved in 50% ethanol (1) against *T. mentagrophytes* (upper photograph) and *T. rubrum* (lower photograph) compared to 50 % ethanol as control (2) and H₂O as control (3).
Figure 3: Analysis of the metabolic content of tomato waste stream extracts after fractionation as described in example 3 by means of TLC. Fractions on the TLC plate were: chloroform fraction (1) ethyl acetate fraction (2) and unfractionated aqueous tomato waste stream extracts (3 and 4). Bands were visualized at 254 nm (A), at 366 nm after derivation with p-anisaldehyde-sulfuric acid (B) and at white light after derivation with p-anisaldehyde-sulfuric acid (C).

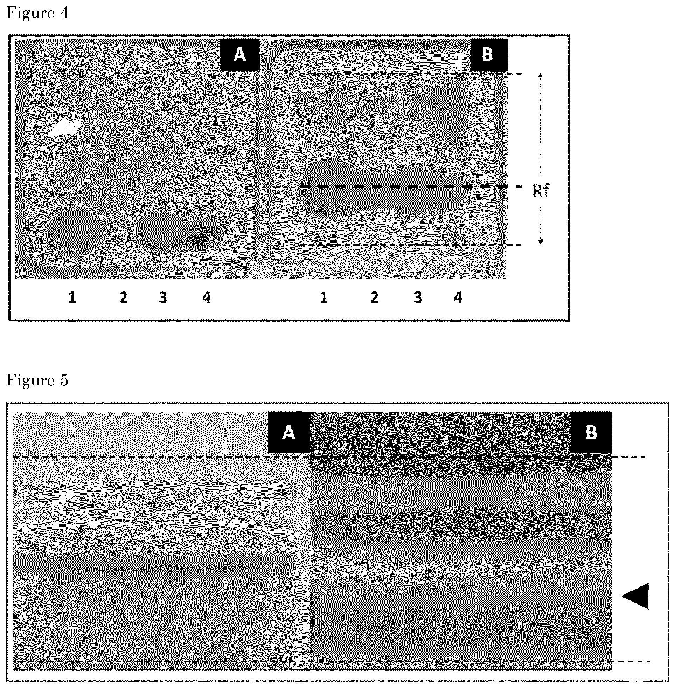

Figure 4: Bioautography antifungal activity of tomato waste stream fractions according to example 3. The TLC plates were loaded with chloroform fraction (1) ethyl acetate fraction (2) and unfractionated aqueous tomato waste stream extracts (3 and 4) and developed with chloroform:acetone:formic acid (75:16,5:8,5). The anti-fungal activity was tested on agar plates containing spores of *T. mentagrophytes* before (A) and after TLC development (B). The Rf value of the inhibition zone after development was calculated by the distance travelled by the inhibition zone divided by the distance travelled by the solvent front.
Figure 5: Developed TLC plate with the active chloroform fraction band indicated with the arrow. Plates were developed with chloroform:acetone:formic acid (75:16,5:8,5) and visualized at 254 nm (A) and 366 nm (B). The active chloroform band (arrow) was scraped from the plate for GC/MS analysis.

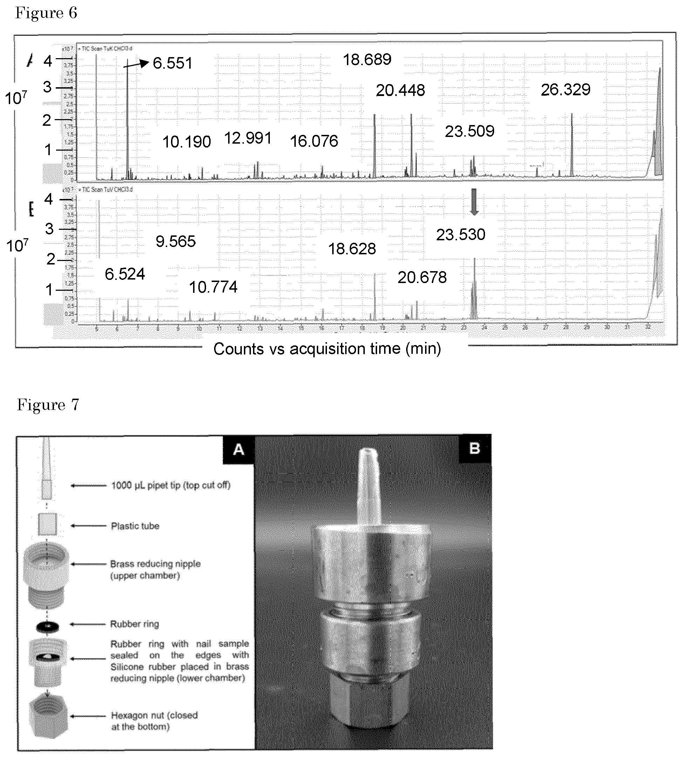

Figure 6: GC/MS profiles of active chloroform fraction isolated from tomato waste streams obtained after a 30 minutes boiling period (A) and after a freeze-thaw cycle (B) both as described at example 3. The arrow indicates the GC/MS peak corresponding with sebacic acid which was more abundant in the fraction with the highest anti-fungal activity
Figure 7: Device for measuring nail penetration with (A) a schematic overview of the components and (B) a photograph of the device.

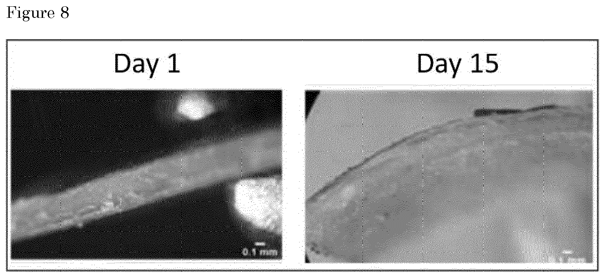

Figure 8: Dorsal overview of the nail clipping used in the manifold as described in Figure 7 with on top of the nail clipping a brilliant blue- dye dissolved in NADES solution at day 1 (starting day) and after 15 days of incubation. The blue dye has been fully penetrated through the nail. The Photographs were made of 40-fold magnification with a Zeiss microscope.

### Detailed description

### Definitions

The term "or" as used herein is defined as "and/or" unless specified otherwise.

The term "a" or " an" as used herein is defined as "at least one" unless specified otherwise.

The term "substantial(ly)" or "essential(ly)" is generally used herein to indicate that it has the general character or function of that which is specified. When referring to a quantifiable feature, these terms are in particular used to indicate that it is for at least 75 %, more in particular at least 90 %, even more in particular at least 95 % of the indicated feature.

In the context of the present application, the term "about" means generally a deviation of 15% or less from the given value, in particular a deviation of 10% or less, more in particular a deviation of 5% or less.

When referring to a noun in the singular, the plural is meant to be included, unless it follows from the context that it should refer to the singular only.

The term "food crop residue" refers to residue, or waste stream of a food crop that is typically left after harvesting of the crop from the plant, typically after harvesting a fruit, nut, grain or tuber, from the plant. Included in the term food crop residue are fractions of the residue, e.g. comprising part of the waste stream, e.g. only the roots, leaves, stems or waste crops of a food crop, mixtures thereof and essentially the entire residue of the food crop after harvesting. Usually, a food crop residue contains at least one of a root, a stem, a leaf or a waste crop of said food crop. The term "food crop" refers herein to a crop that is edible for human beings. Examples of food crops include tomato, egg-plant, potato and pepper, such as bell pepper and chili pepper.

With the term waste crop as used herein is meant a food crop, such as a fruit, nut, tuber, or grain that is not harvested, typically because it is not suitable for sale, e.g. because the fruit is damaged, discolored, misshaped or the like.

The term "polar" as used herein refers to a solvent that has a polarity index of 4 or more, as determinable by the method described in Snyder et al. (Snyder, 1974. J. Chrom. A 92:223-230). Examples of polar solvents include methanol (polarity index of 5.1), ethanol (polarity index 4.3) and water (polarity index 10.2). The term "non-polar" as used herein refers to a solvent that has a polarity index of less than 4. Examples of non-polar solvents include heptane (polarity index of 0.1), hexane (polarity index of 0.1) and toluene (polarity index of 2.4).

With the term "purify" as used herein is meant removing at least one impurity from a crude mixture comprising a target compound and one or more impurities. Typically, the weight fraction of the target compound is increased in the (crude) mixture after one or more purification steps are employed.

The term "retention factor (Rf)" is generally known in the art to describe polarity of a compound. The retention factor is determinable by calculating the ratio of the distance a compound or a mixture of compounds travelled on a thin layer chromatography plate from the starting point, to the total distance the solvent front moved from the starting point. As the skilled person is aware, in order to visualize one or more (target) compound(s) in order to allow determination of their Rf value, it may be required to use a stain, such as p-anisaldehyde.

### Plant extract for use in treating fungal infections

### Food crop residue

Food crops such as tomato plants are typically grown to harvest their fruits, nuts, grains or tubers for the food industry. Although some food crops can be grown and harvested for several years, many food crops are harvested only once. After harvesting their crop the remainder of the plant, including the roots, leaves, stems and waste crops thereof, is typically disposed as agricultural waste.

The inventors surprisingly realized that one or more valuable components exhibiting antifungal activity can be obtained from the waste of a food crop of the family Solanaceae. These one or more components may advantageously be used as organic antifungal agent in various applications, including pharmaceutical applications and applications as disinfectants.

Accordingly, the invention relates to a plant extract from a food crop residue of the family Solanaceae for use in a method of treatment of a fungal infection. The invention further relates to a use of a plant extract as defined herein as a disinfectant and to an object treated with a plant extract as defined herein, preferably wherein the object is a shoe, a sock, clothing or a surface

The plant extract for use according to the invention can in principle be any plant extract derivable from a food crop residue of the family Solanaceae, which exhibits antifungal activity.

Preferably, said food crop residue comprises one or more, preferably two or more, more preferably three or more of a root, a leaf, a stem or a waste crop of a food crop, most preferably said food crop residue comprises a mixture of roots, leaves, stems and waste crops of a food crop. More preferably, said food crop residue contains one or more, preferably two or more of a root, a leaf or a stem of a food crop, most preferably said food crop residue contains a mixture of roots, leaves or stems of a food crop.

A preferred food crop residue comprises between 0-100 wt.% of roots, preferably between 5-50 wt.%, more preferably between 10-20 wt.% of roots, between 0-100 wt.% of leaves, preferably between 10-75 wt.% of leaves, more preferably between 20-50 wt.% of leaves, between 0-100 wt.% of stems, preferably between 20-80 wt.% of stems, more preferably between 30-60 wt.% of stems and between 0-100 wt.% if waste fruit, preferably between 2-20 wt.% of waste crops, more preferably between 5-10 wt.% of waste crops of a food crop of the family Solanaceae, based on the total weight of the food crop residue.

Preferably, said food crop residue comprises at most 5 wt.% of waste food crop more preferably at most 4 wt.%, at most 3 wt.%, at most 2 wt.% at most 1 wt.% of the waste food crop, based on the total weight of the food crop residue. More preferably, said food crop and accordingly said plant extract is essentially free of (an extract from) the fruits, nuts, tubers or grains of a food crop.

Preferably the food crop residue of the family Solanaceae is used within about 7 days, preferably between about 0 and about 5 days, more preferably between about 1 and about 2 days, after harvesting of the food crop from the plant, for the preparation of a plant extract for use according to the invention. In other words, the food crop residue is preferably relatively fresh, meaning it has not started to decompose or grow mold.

Alternatively, the food crop residue of the family Solanaceae may be dried before a plant extract for use according to the invention is prepared therefrom.

Said food crop is preferably organically grown, essentially free of (chemical) pesticides, to avoid contamination of pesticides or other undesired by-products in the plant extract for use according to the invention.

The family of Solanaceae, also referred to as nightshades, is a family of flowering plants belonging to the order Solanales.

Said food crop of the family Solanaceae may in principle be any food crop from the Solanaceae family, but is preferably a food crop from the Solanoideae subfamily. More preferably, said food crop is a food crop from the Genus *Solanum* or the genus *Capsicum,* even more preferably from the genus *Solanum.*

Most preferably, said food crop is a food crop from the genus *Solanum* subgenus *Leptostemonum,* even more preferably from section *Melongena* or *Solanum* subgenus *Solanum sensu stricto,* even more preferably from subgenus *Solanum sensu stricto* section *Lycopersicon* or section *Petota.*

Examples of species from the section *Melongena* include *Solanum aculeastrum, Solanum campechiense, Solanum carolinense, Solanum cataphractum, Solanum citrullifolium, Solanum dimidiatum, Solanum elaeagnifolium, Solanum heterodoxum, Solanum incanum, Solanum linnaeanum, Solanum macrocarpon, Solanum marginatum, Solanum melongena, Solanum rostratum, Solanum sisymbriifolium* and *Solanum virginianum.*

Examples of species from the section *Lycopersicon* include *Solanum arcanum, Solanum chilense, Solanum corneliomulleri, Solanum huaylasense, Solanum peruvianum, Solanum cheesmaniae, Solanum chmielewskii, Solanum galapagense, Solanum habrochaites, Solanum lycopersicum, Solanum neorickii, Solanum pennellii* and *Solanum pimpinellifolium.*

Examples of species from the section *Petota* include *Solanum albornozii, Solanum bulbocastanum, Solanum bukasovii, Solanum burtonii, Solanum cardophyllum, Solanum chilliasense, Solanum commersonii, Solanum demissum, Solanum jamesii, Solanum minutifolium, Solanum paucijugum, Solanum phureja, Solanum pinnatisectum, Solanum refularifolium, Solanum stoloniferum, Solanum stenotomum, Solanum ternatum* and *Solanum tuberosum.*

Examples of species from the genus *Capsicum* include *Capsicum annuum, Capsicum baccatum, Capsicum benoistii, Capsicum buforum, Capsicum caatingae, Capsicum caballeroi, Capsicum campylopodium, Capsicum carassense, Capsicum cardenasii, Capsicum ceratocalyx, Capsicum chacoense, Capsicum chinense, Capsicum coccineum, Capsicum cornuturra, Capsicum dimorphum, Capsicum dusenii, Capsicum eshbaughii, Capsicum eximium, Capsicum flexuosum, Capsicum friburgense, Capsicum frutescens, Capsicum galapagoense, Capsicum geminifolium, Capsicum havanense, Capsicum hookerianum, Capsicum hunzikerianum, Capsicum lanceolatum, Capsicum leptopodum, Capsicum longidentatum, Capsicum longifolium, Capsicum lycianthoides, Capsicum minutiflorum, Capsicum mirabile, Capsicum mosticum, Capsicum neei, Capsicum parvifolium, Capsicum pereirae, Capsicum pubescens, Capsicum piuranum, Capsicum praetermissum, Capsicum ramosissimum, Capsicum recurvatum, Capsicum regale, Capsicum rhomboideum, Capsicum schottianum, Capsicumscolnikianum, Capsicum spina-alba, Capsicum stramoniifolium, Capsicum tocarii* and *Capsicum vilosum.*

In a preferred embodiment, said food crop is selected from *Solanum tuberosum, Solanum lycopersicum, Solanum melongena* and *Capsicuum annuum.* Most preferably said food crop is *Solanum lycopersicum.*

### Components present in plant extract with fungal activity

The inventors have made substantial effort in characterizing the plant extract for use according to the invention, in particular in characterizing one or more antifungal component(s) comprised therein.

The inventors realized that a plant extract with good antifungal properties was characterized with at least one component having a retention factor (Rf) value of between about 0.3 and about 0.35 as determinable with thin layer chromatography, using silica gel 60 F254 as stationary phase and a mixture of chloroform, acetone and formic acid in a (v/v) ratio of 75:16.5:8.5 as liquid phase.

Accordingly, in a preferred aspect, the invention relates to a plant extract for use according to the invention, wherein said extract comprises at least one compound that is characterized by a spot with a retention factor (Rf) value of between about 0.3 and about 0.35 as determinable with thin layer chromatography and using silica gel 60 F254 as stationary phase and a mixture of chloroform, acetone and formic acid in a (v/v) ratio of 75:16.5:8.5 as liquid phase.

The inventors further analysed various plant extracts with different antifungal properties with gas chromatography-mass spectrometry and realized that the concentration of sebacic acid was much higher in a plant extract with relatively high antifungal activity, compared to a plant extract with relatively low antifungal activity.

Sebacic acid is a linear hydrocarbon having terminal carboxylic acid groups, with the chemical formula (CH₂)₈(CO₂H)₂ as depicted in Formula I.

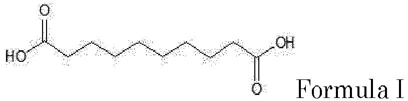

Accordingly, the invention further preferably relates to a plant extract for use according to the invention, comprising sebacic acid or a salt thereof. Further, the invention relates to sebacic acid or a salt thereof for use in the treatment of a fungal infection.

Said salt is preferably a pharmaceutically acceptable salt, such as a sodium salt, a potassium salt or lithium salt.

Preferably, said plant extract for use according to the invention, comprises between about 0.005 and about 95 wt.% of sebacic acid or a salt thereof, more preferably between about 0.05 and about 90 wt.%, even more preferably between about 0.5 and about 70 wt.% of sebacic acid or a salt thereof, between about 1 wt.% and about 60 wt.%, between about 2 wt.% and about 50 wt.%, such as between about 3 wt.% and about 40 wt.%, between about 4 wt.% and about 30 wt.%, between about 5 wt.% and about 25 wt.%, between about 6 wt.% and about 20 wt.%, between 7 wt.% and about 15 wt.%, between about 8 wt.% and about 12 wt.%, based on the total dry weight of the plant extract.

### Medical and non-medical use

The inventors surprisingly realized that a plant extract from food crop residue of the family Solanaceae is effective in the treatment of fungal infections. In particular, excellent efficacy against *Trichophyton* was observed, as demonstrated in the Examples.

*Trichophyton* is a dermatophyte, a group of fungi that infect keratinous tissues such as the skin - in particular the epidermis, hair and nails.

Therefore, *Trichophyton* is capable of causing a number of fungal infections, including but not limited to tinea capitis (also referred to as scalp ringworm), tinea faciei, tinea barbae, tinea corporis (also referred to as ring worm), tinea manuum, tinea unguium (also known as onychomycosis), tinea cruris (also referred to as jock itch) and tinea pedis (also referred to as athlete's foot).

Accordingly, the invention preferably relates to a plant extract from a food crop residue of the family Solanaceae or sebacic acid or a salt thereof for use in a method of treatment of tinea capitis, tinea faciei, tinea barbae, tinea corporis, tinea manuum, tinea unguium, tinea cruris and tinea pedis. Preferably, the invention relates to the plant extract as defined herein or sebacic acid or a salt thereof for use in a method of treatment of tinea unguium, also referred to as onychomycosis. More preferably the invention relates to the plant extract as defined herein or sebacic acid or a salt thereof for use in a method of treatment of distal and lateral subungual onychomycosis.

Preferably, said plant extract or sebacic acid or salt thereof is for use in a method of treating a fungal infection caused by *Trichophyton,* more preferably one or more of *T. concentricum, T. equinum, T. erinacei, T.megninii, T.mentagrophytis, T. interdigitale, T. rubrum, T. schoenleinii, T. simii, T. soudanense, T. tonsurans, T. verrucosum, T. violaceum, T. yaoundei,* more preferably *T. rubrum* and/or *T. mentagrophytis.*

Said treatment comprises administration of said plant extract or sebacic acid or a salt thereof to a subject in need thereof. Said subject may be any subject suffering from a fungal infection. Preferably, said subject is an animal, preferably a mammal, including a human. Preferably, said subject is a human, a pet animal such as a cat, a dog, a guinea pig, a rat, a mouse, a hamster, a Guinea pig, a ferret or a rabbit or a livestock animal such as a horse, a sheep, a goat, a cow, a pig, a donkey, a llama or an alpaca. Most preferably the subject is a human.

Said plant extract may be administered systemically, such as orally, but is preferably administered locally, such as topically. This is advantageous, because local administration is typically associated with fewer side-effects, as the active principle is administered directly to the infected area. Accordingly, in a preferred embodiment of the invention, the plant extract as defined herein or sebacic acid or a salt thereof is administered to or near an area that is infected. Preferably the plant extract as defined herein or sebacic acid or a salt thereof is administered topically.

The invention also relates to a method of treating a fungal infection, preferably a fungal infection caused by *Trichophyton,* more preferably *T. rubrum* and/or *T. mentagrophytis,* comprising administering a plant extract from a food crop residue of the family Solanaceae or sebacic acid or a salt thereof to a subject in need thereof. Said subject is as defined herein above. Said administration preferably comprises local administration, preferably topical administration, as described herein above.

The invention further relates to a use of a plant extract from a food crop residue of the family Solanaceae or a use of sebacic acid or a salt thereof for the manufacture of a medicament for the treatment of a fungal infection, preferably a fungal infection caused by *Trichophyton,* more preferably *T. rubrum* and/or *T. mentagrophytis.* Said subject is as defined herein above. Said administration preferably comprises local administration, preferably topical administration, as described herein above.

The invention further relates to a use of a plant extract as defined herein, sebacic acid or a salt thereof as a disinfectant.

The invention further relates to an object treated with an extract as defined herein, sebacic acid or a salt thereof, or a composition as defined herein. Said object is preferably a shoe, a sock, clothing or a surface, such as the surface of a table or the like.

### Composition

The invention further relates to a composition comprising a plant extract from a food crop residue from the Solanaceae family as defined herein or sebacic acid and a suitable carrier.

Said suitable carrier may be any carrier that is compatible with said plant extract or sebacic acid. Preferably, said suitable carrier is dermatologically approved.

Said composition is preferably for use in treatment of a fungal infection, preferably Trichophyton, more preferably *T.mentagrophytis* or *T.rubrum,* more preferably for use in the treatment of onychomycosis, as described herein above in the section `medical and non-medical use'. Alternatively, said composition is suitable to be used as a disinfectant as described herein above in the section 'medical and non-medical use'.

Said suitable carrier is preferably a dermatologically approved carrier, more preferably a Natural Deep Eutectic Solvent (NADES).

The inventors surprisingly realized that the efficacy of the plant extract as defined herein may be markedly increased when it is dissolved or dispersed in a NADES. NADES are formed by combinations of two or more organic molecules such as sugars, poly-alcohols, amino acids, organic acids, and bases, which in certain molar ratios, can form a liquid at room temperature, i.e., with a melting point much lower than those of its individual components (Mustafa et al. 2021).

NADES advantageously forms a sustainable solvent, posing practically no environmental hazards.

Without wishing to be bound by any theory, it is believed that the NADES enables one or more active components, such as sebacic acid or a salt thereof, present in the plant extract to penetrate through keratinous tissue, such as a nail, hair or the epidermis to reach the infected area. In many fungal infections, in particular those caused by *Trichophyton,* the fungus invades tissue behind a layer of keratinous tissue. Therefore, these fungal infections are often difficult to treat, as the keratinous tissue may serve as a barrier layer between the fungus and the active component. This is in particular a problem in the treatment of onychomycosis, more preferably distal and lateral subungual onychomycosis, as nails are thick and hard structures that are difficult to pass by many antifungal agents.

Preferably, said NADES comprises one or more, preferably two or more, more preferably three or more, in particular four or more components selected from 1,2-propanediol, 1,3-butanediol, sodium acetate, aconitic acid, adonitol, α-L-rhamnose, β-alanine, betaine, choline chloride, citric acid, D-(-)-fructose, D-(+)-galactose, D-(+)-glucose, D-(+)-trehalose, DL-malic acid, D-mannose, D-proline, D-sorbitol, D-xylose, glycerine, glycol, glycolic acid, glycine, inositol, L-(+)-tartaric acid, lactic acid, 1-glutamic salt, L-proline, L-serine, malic acid, malonic acid, maltose, meso-erythritol, oxalic acid, phytic acid sodium, proline, propylene glycol, quercetin, raffinose, ribitol, sorbose, sucrose, tri-ethylene glycol, urea and xylitol, more preferably said NADES comprises one or more of urea and lactic acid.

More preferably, said NADES is selected from mixtures of (i) urea, glucose and citric acid; (ii) lactic acid and sodium acetate; (iii) lactic acid, glycine and water; (iv) lactic acid and beta-alanine; (v) glycolic acid and betaine; (vi) urea, lactic acid, propylene glycol and glycerol; (vii) urea, lactic acid and propylene glycol; (viii) urea and proline; (ix) urea and glycerin; (x) urea and betaine. Most preferably, said NADES is selected from one or more of mixtures (i) to (vi).

Preferably, the molar ratio between the individual components of the NADES is between 1:1(: 1) and (1:)1:10, more preferably between (1:)1:2 and (1:)1:5.

Accordingly, the invention preferably relates to a composition comprising a plant extract as defined herein and a NADES selected from a NADES having the composition urea, glucose and citric acid in a molar ratio of about 1:1:1, lactic acid, sodium acetate in a molar ratio of about 3:1, lactic acid, glycine and water in a molar ratio of about 3:1:3, lactic acid and beta-alanine in a molar ratio of about 1:1, glycolic acid and betaine in a molar ratio of about 2:1, urea, lactic acid, propylene glycol and glycerol in a molar ratio of about 5:1:1:3, urea, lactic acid and propylene glycol in a molar ratio of about 1:1:4, urea, lactic acid and propylene glycol in a molar ratio of about 3:1:9, urea and proline in a molar ratio of about 1:1, urea and glycerin in a molar ratio of about 1:3, urea and betaine in a molar ratio of about 1:1.

A composition comprising a plant extract as defined herein or sebacic acid and a NADES are particularly suitable for treatment of fungal infections, preferably a fungal infection caused by *Trichophyton,* more preferably *T. rubrum* and/or *T. mentagrophytis,* even more preferably fungal nail infections, in particular onychomycosis.

Alternatively or additionally, said suitable carrier is preferably one or more of water, ethanol, propanol or isopropanol, propylene glycol, butylene glycol, dipropylene glycol, ethoxylated or propoxylated diglycols, cyclic polyols, petrolatum and vegetable oil.

Such a composition according to the invention is particularly suitable for treatment of fungal infections, preferably a fungal infection caused by *Trichophyton,* more preferably *T. rubrum* and/or *T. mentagrophytis,* in particular a fungal infection other than fungal nail infections, such as infections of the skin epidermis or hair or suitable for use as a disinfectant.

Said composition according to the invention is preferably formulated as a cream, lotion, balm, shampoo, ointment, nail polish, topical solution, topical suspension, foam, gel or spray. Preferably, the composition for use according to the invention is a nail polish.

Said composition according to the invention may comprise one or more further additives, such as a film-forming resin, a stabilizer, a dispersant, a uv-stabilizer, a plasticizer, a dye, a pigment, a mica, a fragrance, a humectant, an occlusive, a pH adjuster, an emulsifier, an emollient and a preservative.

The invention further relates to a method for preparing a composition according to the invention, comprising contacting a plant extract as defined herein or sebacic acid or a salt thereof with a suitable carrier.

### Method of preparing a plant extract for use according to the invention

Preferably, the plant extract for use according to the invention may be prepared and/or is obtainable by a method, comprising the steps of
a) obtaining an aqueous juice from residue of a food crop belonging to the family of Solanaceae;
b) fractionating said aqueous juice with chromatography using a non-polar stationary phase, preferably comprising a copolymer styrene-divinylbenzene adsorbent resin as stationary phase, to obtain a fractionated juice;
c) drying the fractionated juice to obtain a dried fractionated juice;
d) reconstituting the dried fractionated juice in an organic solvent or organic solvent mixture to obtain a crude extract;
e) removing undissolved solids from the crude extract;
f) removing the organic solvent from the crude extract obtained in step e) to obtain a dried crude extract;
g) reconstituting the dried crude extract in water and a non-water-miscible organic solvent or non-water-miscible organic solvent mixture under vigorous agitation;
h) separating the non-water-miscible organic solvent or non-water-miscible organic solvent mixture from the water; and
i) removing the non-water-miscible organic solvent to obtain the plant extract.

Accordingly, the invention relates to a method for preparing a plant extract comprising at least the steps a)-i). In addition, the invention relates to a plant extract obtainable by the method as defined herein.

Said food crop residue belonging to the family Solanaceae may be derived from any suitable source, but is preferably obtained from an agricultural waste stream. Preferably, said agricultural waste stream is allowed to rest for between about 0 and about 7 days, before it is subjected to step a). More preferably between about 1 and about 5 days, such as between about 2 and about 3 days.

Preferably, said food crop residue is not subjected to a pre-treatment step, in particular a pre-treatment step involving elevated or reduced temperatures and/or chemicals.

Optionally however, said food crop residue may be subjected to a pre-treatment to disrupt the structure of the food crop material and facilitate the process of obtaining an aqueous juice therefrom.

Preferably, such a pre-treatment comprises one or more steps of boiling in the presence of water or subjecting the material to one or more cycles of freeze-thawing. If such pre-treatment comprising a step of boiling, typically said food crop residue is boiled in water for a duration of between 15 and 60 minutes, more preferably between 20 and 40 minutes, such as around 30 minutes.

Preferably step a) is achieved using any method known in the art, for example by squeezing said food crop residue to separate an aqueous liquid having antifungal activity from solid plant residues.

The solid plant residue typically comprises fibers, cellulose, starch and the like. Optionally, said solid waste is squeezed more than once and/or extracted with an aqueous solvent to obtain a further aqueous liquid comprising an antifungal component. Said further aqueous liquid may be combined with the first aqueous liquid, or may be processed separately.

Said aqueous liquid is preferably obtained by squeezing a food crop residue of the family Solanaceae using a mechanical press.

Alternatively or additionally, said aqueous juice may be obtained by grinding a food crop residue of the family Solanaceae and mixing said ground food crop of the family Solanaceae in an aqueous medium, followed by separating the solid residue from the liquid phase.

Said aqueous liquid obtained in step a) may be optionally dried to remove at least a substantial part of the water to obtain a dried juice.

Said drying may be achieved using any suitable means in the art, for example by evaporating the juice, preferably under reduced pressure to remove the aqueous phase or by means of lyophilization. Preferably, said drying is achieved by means of lyophilization.

In step b) said aqueous liquid obtained in step a) or said dried juice is subjected to a step of chromatography to obtain a fractionated juice. Said chromatography may be any suitable method of chromatography known in the art, such as column chromatography, flash column chromatography, high-performance liquid chromatography, or preparative thin layer chromatography (TLC). Preferably, said chromatography is column chromatography or flash column chromatography.

Preferably, said column chromatography is reverse-phase column chromatography. More preferably, said reverse-phase column chromatography comprises a non-polar resin as stationary phase.

Preferably said non-polar resin is a non-polar copolymer styrene-divinylbenzene adsorbent resin, such as Diaion^{®} HP-20, a silica-bound C18 (octadecyl), C8 (octyl), C4 (butyl), cyclohexyl or phenyl resin.

Preferably, said reverse-phase column chromatography comprises a polar liquid phase, more preferably a mixture of ethanol and water as a liquid phase.

In a specific embodiment, said aqueous liquid obtained in step a) or said dried juice is loaded onto an upper part of the column filled with non-polar resin, preferably a non-polar copolymer styrene-divinylbenzene adsorbent resin, more preferably Diaion^{®} HP-20, which is subsequently washed with water, followed by elution with a substantially polar solvent or solvent mixture other than water, preferably 90% ethanol in water, 70% ethanol in water, ethanol, methanol, acetonitrile or acetone.

Alternatively, said said aqueous liquid obtained in step a) or said dried juice is thoroughly mixed with a non-polar copolymer styrene-divinylbenzene adsorbent resin, more preferably Diaion^{®} HP-20 in water and said non-polar copolymer is subsequently loaded in a column. Said resin is preferably washed with water followed by elution with a substantially polar solvent or solvent mixture other than water, preferably 90 vol.% ethanol in water, 70 vol.% ethanol in water, ethanol, methanol, acetonitrile or acetone.

Preferably said substantially polar solvent or solvent mixture is a gradient from about 0 vol.% to about 90 vol.% or about 0 to about 70 vol.% ethanol in water.

The amount of resin, water and polar solvent is dependent on the amount of aqueous juice obtained in step a) or a dried aqueous juice, to be purified. It is within the ability of the skilled person to select appropriate amounts of resin, water and polar solvent, based on his common general knowledge and the information provided herein.

As a rule of thumb, typically the ratio of dried aqueous juice and resin is about 8-15 mL of resin per 1 g of dried juice, preferably between 10 and 14 ml of resin per 1 g of juice.

As a rule of thumb, typically the ratio between resin and eluent is between about 1-5 mL of eluent per 1 mL of resin, preferably between about 2-3 mL of eluent per 1 mL of resin.

The polar solvent fractions comprising fungal activity are subsequently combined and dried to obtain a dried fractionated juice. Said dried fractionated juice is reconstituted in an organic solvent or organic solvent mixture as defined in step d).

Said organic solvent or solvent mixture is an organic solvent or solvent mixture that has the ability to at least substantially dissolve the one or more compounds having antifungal properties.

Accordingly, said organic solvent or solvent mixture should preferably have a polarity index of between about 2 and about 7, preferably between about 3 and about 6, more preferably about 4.

Typically, when an organic solvent mixture is used, two or more organic solvents are used that are miscible with one another. Typically, one of the solvents has a relatively high polarity index, preferably of 4 or more, preferably 4.5 or more, even more preferably 5 or more. Typically one of the solvents has a relatively low polarity index, preferably 3 or less, preferably 2 or less, more preferably 1 or less.

Herein, the polarity index of a solvent or solvent mixture is determinable by the method described in Snyder et al. (Snyder, 1974. J. Chrom. A 92:223-230).

When preparing an organic solvent mixture suitable for reconstituting the dried juice in step d) two or more organic solvents are mixed to obtain a solvent mixture having a polarity index within the indicated range.

Examples of organic solvents with a relatively high polarity index suitable in the method described herein include ethyl acetate, methyl n-propyl ketone, methyl ethyl ketone, 1,4-dioxane, acetone, methanol, ethanol, pyridine, 2-methoxyethanol, acetonitrile, propylene carbonate, N,N-Dimethylformamide, dimethyl acetamide, N-Methylpyrrolidone and dimethyl sulfoxide

Examples of organic solvents with a relatively low polarity index suitable in the method described herein include pentane, 1,1,2-trichlorotrifluoroethane, cyclopentane, heptane, hexane, iso-octane, petroleum ether, cyclohexane, n-butyl chloride, toluene, methyl t-butyl ether, o-xylene, chlorobenzene, o-dichlorobenzene, (di)ethyl ether, dichloromethane, ethylene dichloride, n-butyl alcohol, isopropyl alcohol, n-butyl acetate, isobutyl alcohol, methyl isoamyl ketone, n-propyl alcohol, tetrahydrofuran, chloroform, methyl isobutyl ketone.

Preferably, said organic solvent or solvent mixture is acetone or a mixture of acetone and hexane, preferably in a ratio of about 3 to 1 to 7 to 1, more preferably 5 to 1.

The amount of organic solvent or solvent mixture to be used is dependent on the amount of dried fractionated juice to be purified. It is within the ability of the skilled person to select appropriate amounts of organic solvent or organic solvent mixture and dried fractionated juice, based on his common general knowledge and the information provided herein.

As a rule of thumb, typically the ratio of dried fractionated juice and organic solvent or organic solvent mixture is about 0.5-5 mL of organic solvent or organic solvent mixture per about 1 g dried fractionated juice, preferably about 1-2 mL of organic solvent or organic solvent mixture per about 1 g of dried fractionated juice.

Subsequently, any undissolved solids are removed from the crude extract (step e). Removing undissolved solids may be achieved using any suitable means known in the art, for example by means of filtration over a suitable filter such as a Büchner filter, a paper filter or over a membrane, optionally under reduced pressure, or by means of decantation of the fluids from the undissolved solids.

After removal of any undissolved solids, said organic solvent or solvent mixture is removed to obtain a dried crude extract (step f). Preferably, said organic solvent or organic solvent mixture is removed by evaporation, optionally under reduced pressure.

Said dried crude extract obtained in step f) is subsequently reconstituted in water and a non-miscible organic solvent or non-miscible organic solvent mixture under vigorous agitation. The non-miscible organic solvent or non-miscible organic solvent mixture may in principle be any organic solvent or organic solvent mixture that is not miscible with water and that at least partly solubilizes the one or more compounds with antifungal properties present in the plant extract.

Preferably, the non-water-miscible organic solvent or organic solvent mixture is a solvent with a polarity index of between 0.1 and 4.5, preferably between 1 and 4.3, more preferably between 2 and 4.1.

Preferably, the non-water-miscible organic solvent or non-water-miscible organic solvent mixture is selected from one or more of pentane, 1,1,2-trichlorotrifluoroethane, cyclopentane, heptane, hexane, iso-octane, petroleum ether, cyclohexane, n-butyl chloride, toluene, methyl t-butyl ether, o-xylene, chlorobenzene, o-dichlorobenzene, (di)ethyl ether, dichloromethane, ethylene dichloride, n-butyl alcohol, isopropyl alcohol, n-butyl acetate, isobutyl alcohol, methyl isoamyl ketone, n-propyl alcohol, tetrahydrofuran, chloroform, methyl isobutyl ketone, 1,2-dichloroethane, dimethyl formamide, ethyl acetate, 2,2,4-trimethylpentane or a mixture thereof.

More preferably, said non-water-miscible solvent or solvent mixture is chloroform, dichloromethane or a mixture thereof.

Said agitation may be achieved using any suitable method known in the art, such as by shaking, stirring or swirling. Typically, said water and non-miscible organic solvent or solvent mixture is agitated for a time sufficient to allow distribution of water-soluble components into the water phase and organic solvent-soluble components into the non-miscible organic water phase. Preferably, the mixture of water and non-miscible organic solvent is vigorously agitated for between 1 second and 1 minute, such as between 5 seconds and 30 seconds, in particular about 20 seconds.

Typically, the components that become dissolved in the water phase are relatively polar, whereas the components soluble in the organic solvent phase are relatively non-polar.

The amount of water and non-miscible organic solvent or solvent mixture is dependent on the amount of crude extract to be reconstituted. It is within the ability of the skilled person to select appropriate amounts of water and non-miscible organic solvent or organic solvent mixture, based on his common general knowledge and the information provided herein.

As a rule of thumb, typically the concentration of crude extract in solvent is about 0.001 to about 10 mg/mL, preferably about 0.05 to about 5 mg/mL, such as around 0.1-1 mg/mL.

After said vigorous agitation, said water and non-water-miscible organic solvent or solvent mixture are separated. This is usually achieved by allowing the two phases to phase-separate. The two phases may accordingly be separated using a suitable method or means, for example by decantation or using an extraction funnel.

Optionally, the water phase obtained in step h) is washed at least once with a non-miscible organic solvent or non-miscible organic solvent mixture. Preferably, said water phase is washed with the same non-miscible organic solvent mixture as used in step g). Said washings may be combined with the non-water-miscible organic solvent or solvent mixture obtained in step g) or may be processed separately.

Optionally, said non-miscible organic solvent or non-miscible organic solvent mixture may be treated with a water-absorbing agent, to ensure removing essentially all water and moisture therefrom before further processing the non-miscible organic solvent. Examples of such water-absorbing agents include Na₂SO₄ and MgSO₄.

If such a drying agent is used, it may be removed from the non-miscible organic solvent or solvent mixture using a suitable method for removing solids from liquids, for example by filtration.

Said non-miscible organic solvent or solvent mixture obtained in step h) and optionally said non-miscible organic solvent or solvent mixture washings are subsequently removed to obtain the plant extract with antifungal properties.

Said non-miscible organic solvent or non-miscible organic solvent mixture may be removed using any suitable method known in the art, typically by means of evaporation, optionally under reduced pressure.

Said dried plant extract may optionally be left to dry, preferably under reduced pressure to remove trace amounts of non-miscible organic solvents or non-miscible organic solvent mixture.

Optionally, said dried plant extract may be subjected to one or more further purification steps, to isolate one or more antifungal components therefrom.

Preferably, the plant extract obtained in step i) is further subjected to one or more purification steps, preferably comprising a step of chromatography.

Said chromatography may be column chromatography, flash column chromatography, high-performance liquid chromatography, or preparative thin layer chromatography (TLC). Preferably, said chromatography is preparative TLC.

Preferably, said chromatography uses silica gel 60 F254 as stationary phase and a mixture of chloroform, acetone and formic acid in a (v/v) ratio of 75:16.5:8.5 as liquid phase, wherein at least one compound having an Rf value of between about 0.3 and about 0.35 is isolated from the chromatography.

In the method for preparing a plant extract for use according to the invention, the skilled person may analyse obtained fractions and intermediate products at any time using TLC, for example using the conditions as specified herein. The TLC plate may be analysed under uv light (254 nm and/or 366 nm or white light). Optionally, the TLC plates may be treated with one or more suitable stains for development of TLC plates. Preferably, said TLC plate is developed using p-anisaldehyde-sulfuric acid stain.

As the skilled person will appreciate, other conditions may also be employed to purify the plant extract for use according to the invention, provided that one or more components with antifungal properties, in particular having an Rf value of between about 0.3 and about 0.35, using silica gel 60 F254 as stationary phase and a mixture of chloroform, acetone and formic acid in a (v/v) ratio of 75:16.5:8.5 as liquid phase, is separated from one or more impurities.

The skilled person is capable of selecting suitable conditions for purification, based on his common general knowledge and the information provided herein. In particular, the skilled person may use the retention factor of the at least one compound, using the stationary and liquid phases as described herein.

During the method of preparing a plant extract, preferably the method steps are performed at a temperature of at most 90 °C, preferably at most 80 °C, more preferably at most 50 °C, in particular at a temperature of between about 0 and about 50 °C. Advantageously, keeping the temperature as low as possible reduces the risk of temperature-induced degradation of one or more components present in the extract, in particular one or more components having antifungal properties.

For the purpose of clarity and a concise description, features are described herein as part of the same or separate embodiments, however, it will be appreciated that the scope of the invention may include embodiments having combinations of all or some of the features described.

The invention is demonstrated by the following examples.

### Examples

### Example 1: Methods for making a plant extract from food crop residue of tomato plants.

### Step 1: Preparing an aqueous juice from tomato plant residue

### Method A:

An aqueous juice was obtained after freeze-thawing of freshly harvested tomato plant parts (including roots, stems and leaves of the plant) followed by squeezing the freeze-thawed tomato plant under mechanical pressure.

### Method B:

An aqueous juicy substance was obtained after 30 minutes of boiling of freshly harvested tomato plant parts (including roots, stems and leaves of the plant) in water followed by squeezing the boiled tomato plant under mechanical pressure.

### Step 2: Obtaining a fraction with antifungal activity from the aqueous juice

The aqueous juice obtained by any one of methods A-B was purified on a non-polar copolymer styrene-divinylbenzene adsorbent resin type Diaion^{®} HP-20. Good results were obtained by freeze-drying the aqueous juice from method A or B and subsequently dissolving 10 gram of freeze dried material in 1 litre of water. After adding 120 mL of said resin, the mixture was shaked for 16 hours in a 3 litre Erlenmeyer at 120 rpm at 4 °C. Thereafter the mixture was filtered through a 3MM Whatman filter to separate the unbound material from the resin. The resin was washed 6 times with 0.5 Litres of water and the resin was poured into a glass column. The resin was eluted with 250 mL 70% ethanol to elute the fraction with high anti-fungal activity from the resin.

### Example 2: A bioassay for testing the antifungal activity of plant extracts against T. mentagrophytis and T. rubrum based on microtiter plate assays.

The antifungal activity against *Trichoderma mentagrophytis* and *Trichoderma rubrum* was tested both in microtiter plate assay test and in a spot inhibition test. Fungal strains of *Trichophyton mentagrophytes* and *Trichoderma rubrum* (strain numbers CBS 388.58 and CBS 389.58 respectively) were obtained from Centraalbureau voor Schimmelcultures (CBS) from the Westerdijk Fungal Biodiversity Institute (Utrecht, the Netherlands). Culturing was routinely performed on CM-agar plates (50 g/L CM0059 Malt Extract Agar plates (Oxoid)) that were aerobically incubated for three days at 30°C and seven days at room temperature in constant light. Spores were harvested using sterile 0.05% Saline Tween (Sigma-Aldrich) made in physiological saline (PS). The spores were filtered through a sterile folded Amplitude EcoCloth Wipe (VWR International), and 0.05% Saline Tween was added until a volume of 30 mL was reached. The suspensions were centrifuged for 10 minutes at 3000 rpm at 5°C and the supernatant was discharged. Next, 1 mL of PS was added to the pellet and after vortexing, the spores were counted using a TC20 Automated Cell Counter (3-8 µm set for cell size). The spores were stored at 4°C and used within two weeks.

For the microtiter plate assay the fractions obtained from Example 1 were first dried and subsequently dissolved in 50% ethanol. 2 µl of each fraction was added to 200 µl CM medium present in the wells of a microtiter plate in the presence 5×10⁴ spores/ml of *Trichoderma mentagrophytis* or *Trichoderma rubrum.* The microtiter plate was subsequently incubated at 30 °C for 18-20 hr. After this incubation the germination of the spores in the presence of the fractions was assessed and compared to controls. 50% ethanol was used as a negative control. The results of this assay are shown in Figure 1.

It can be concluded from Figure 1 that an extract obtained from a tomato waste stream exhibited antifungal activity against both *T. mentagrophytis* and *T.rubrum* compared to controls.

### Example 3: A bioassay for testing the antifungal activity of plant extracts against T. mentagrophytis and T. rubrum based on spot inhibition assay.

For the spot inhibition test 25 ml of 0.7% agar of (45° C) containing 5×10⁶ spores of *Trichoderma mentagrophytis* or *Trichoderma rubrum* was added on CM-agar plates. After solidifying, 5 µl of each tomato extract dissolved in 50% ethanol (1), 50 % ethanol as control (2) and H₂O as control (3) were loaded on the plate as spots. The plates were incubated at room temperature for 20 days. The growth and putative inhibition zone was assessed at different periods during incubation (figure 2)

It can be derived from Figure 2 that a clear inhibition zone was visible around the spot containing the tomato extract, whereas no inhibition was visible at the control treatments.

### Example 4: Isolation and identification of antifungal components from a waste stream from organic cultivated tomato plants.

An aqueous juice obtained from a waste stream obtained with method A was lyophilized. 500 g of the lyophilized product was stirred for 10 minutes with 500 mL hexane:acetone (1:5, v/v) and sonicated for 30 minutes at 40 °C to remove non-relevant compounds and to obtain an extract enriched in components having antifungal activity. The mixture was filtered through a filter paper to separate the soluble fraction from the non-soluble fraction. The non-soluble fraction was reextracted with hexane:acetone (1:5, v/v) to enhance the final yield of antifungal activity. The soluble fractions were pooled and dried using a BÜCHI Rotavapor R-210, ±215 mbar at 30°C until the solvents were evaporated. The dried extract (~1,5 g) was resuspended in 100 mL chloroform:water (1:1, v/v). The mixture was sonicated for 15 minutes until it was completely homogenized. The lower chloroform part was separated from the water fraction and the water fraction was washed with chloroform. The chloroform fractions were pooled. The remaining water fraction was extracted with an equal volume of ethyl-acetate. After complete partitioning of ethyl acetate and water, the lower water fraction was separated from the ethyl acetate. The water fraction was washed two other times with ethyl-acetate and all ethyl-acetate fractions were pooled. Both the pooled chloroform and the pooled ethyl acetate fractions were evaporated to dry and the remaining water fraction was lyophilized. The content of compounds in the dried fractions was analysed by means of Thin Layer Chromatography (TLC).

The procedure for TLC was as follows:
TLC Silica gel 60 F254 plates (20x10 cm and 10x10 cm) (Merck KGaA) were flushed with pure ethanol and dried with a cold air stream. The chloroform and ethyl acetate extracts (5 pL of 10 mg/mL) were applied on the TLC plates using an automatic TLC sampler 4 (CAMAG). The application length of each band was 8 mm and the track distance was set at 22 mm. The mobile phase consisted of chloroform:acetone:formic acid (75:16.5:8.5). The TLC plates were developed until the solvent was migrated 90 mm from the application point. The plates were dried with a cold air stream. The developed plate was analysed with a TLC visualizer with white light, 254 nm, and 366 nm before and after derivatization. Derivatization was conducted with 2 mL of p-anisaldehyde-sulfuric acid reagent (0,5 mL of p-anisaldehyde, 10 mL of acetic acid, 85 mL of methanol, and 5 mL of sulfuric acid) using an automatic CAMAG Derivatizer after which the plates were heated for 3 minutes at 100°C on a TLC plate heater 3 (CAMAG). VisionCats version 2.5 was used to control the CAMAG system. The results are shown in Figure 3.

To determine in which of the TLC bands the biologically active compound(s) were present, bioautography was used. This is a suitable method for target-directed isolation of biological active compounds on a TLC chromatogram, according to the method described in Grzelak et al (2013). The chloroform (1), ethyl acetate (2) and the original unfractionated aqueous juice obtained as described in Example 1 method 1A + step 2 (3) and Example 1 method 1B + step 2 (4) were applied on a TLC plate and the plates were placed with the aluminium side downwards on 12x12 squared Petri dishes containing solid CM-agar. 40 mL of soft CM-agar with 1 × 10⁵ spores/mL of *Trichoderma rubrum* were poured over the TLC plate and allowed to solidify. Plates were grown in constant light at room temperature for two weeks. The TLC separated compounds were assessed for antifungal activity based on the presence of a growth inhibition zone. The growth inhibition zone was only present in the chloroform fraction and not in the ethyl acetate fraction (Figure 4A). After development of the TLC plate the inhibition zone was used to determine the Rf value as a marker for preparative isolation of the biological active compound(s) (Figure 4B).

For preparative isolation of the biological active compound(s) 30 mg/mL of the chloroform fraction was applied on a preparative TLC (PLC plate Silica gel 60 F254; 2 mm, 20x20 cm). The TLC plate was developed using the same solvent system as described above for the TLC plate. The band corresponding with the marked Rf (Figure 5) was scraped from the TLC plate, dissolved in 10 mL ≥99,9% methanol and centrifuged for 15 minutes at 4000 rpm. The supernatants were dried in a CentriVap Benchtop Vacuum Concentrator (LabconcoTM) and used for further GC-MS analyses.

The above was repeated with an extract obtained with Method B as described in Example 1. Notably, it was found that the antifungal activity against *T. rubrum* and *T. mentagroghytes* of chloroform fraction derived from this waste stream was significantly lower: 1 mg/mL extract obtained with method A resulted in an inhibition zone in a spot inhibition assay of 4.15 cm for *T.mentagrophytes* and 3.18 cm for *T.rubrum* whereas the inhibition zones of 1 mg/mL extract obtained with method B were 2.11 cm for *T.mentagrophytes* and 0.42 cm for *T.rubrum.*

The results of the GC-MS analysis of both extracts are shown in Figure 6.

From Figure 6 we can conclude that a peak identified as sebacic acid was highly present in the fraction with the high antifungal activity and lower in the fraction with lower antifungal activity. This compound is correlated with the antifungal activity against *T. rubrum* and *T. mentagrophytes.*

### Example 5: Using Natural Deep Eutectic Solvents (NADES) as a carrier for extracts and biological active compounds penetrating the nail

Several NADES solutions were tested on nail penetration. Nail penetration was tested in two assays. In the first assay the nail penetration was assessed by means swelling after incubation of a human nail clippings in a NADES solution for 24 hours and subsequently drying for 90 minutes in open air. The size of the nail clippings were approximately 0.07 cm² with a thickness of 0.4 mm. Water was used as a control. The results are shown in table 1.

**Table 1: weight increase and weight loss after drying (90 min) of nail clippings after 24 h incubation of various NADES**

| No. | Compositions [molar ratio] | | | | Weight increase (%) | Weight loss after drying (%) |
|---|---|---|---|---|---|---|
| 1 | Urea [1] | Lactic acid [1] | Propylene glycol [4] | N/A | 2 | 0 |
| 2 | Urea [3] | Lactic acid [1] | Propylene glycol [9] | N/A | 1 | 0 |
| 3 | Urea [1] | Proline [1] | N/A | N/A | 3 | 0 |
| 4 | Urea [1] | Glycerine [3] | N/A | N/A | 1 | 0 |
| 5 | Urea [1] | Betaine [1] | N/A | N/A | 4 | 0 |
| 6 | Urea [1] | Glucose [1] | Citric acid [1] | N/A | 12 | 2 |
| 7 | Lactic acid [3] | Sodium acetate [1] | N/A | N/A | 14 | 1 |
| 8 | Lactic acid [3] | Glycine [1] | Water [3] | N/A | 16 | 2 |
| 9 | Lactic acid [1] | Beta-alanine [1] | N/A | N/A | 8 | 1 |
| 10 | Glycolic acid [2] | Betaine [1] | N/A | N/A | 9 | 0 |
| 11 | Urea [5] | Lactic acid [1] | Propylene glycol [1] | Glycerol [3] | 10 | 0 |
| 12 | water | N/A | N/A | N/A | 26 | 14 |

The increase of weight of the nail clippings after incubation in the different NADES varies from 1% to 16% whereas the increase of pure water was 26%. However after drying of the water incubated clippings for a short period of 90 minutes more than 50% of the penetrated water was already released. In contrast to that the nail clippings incubated in NADES show only a very small decrease of the weight after drying indicating that the NADES was penetrated in the nail.

For example, good results were obtained for NADES 11 showing a significant increase of weight after incubation but no weight-loss after drying.

For a second test for nail penetration by NADES a manifold as depicted in Figure 7 was used. A nail punch derived from donkey hoof with a diameter of 0.5 cm and a thickness of 3.5 mm was sealed in the manifold allowing a water-tight connection between the upper and the lower chamber of the manifold. The blue dye "Neelicol Brilliant Blue FCF food dye" (Neelikon Food Dyes & Chemicals Ltd.) was dissolved in NADES 10 at a concentration of 50 mg/mL for visual inspection of the penetration. 500 µL of the NADES dye solution was pipetted in the upper chamber on top of the hoof. Penetration was followed for 10 day and assessed by the migration distance of the blue dye through the nail. The results are shown in Figure 8.

It can be derived from Figure 8 that the blue dye dissolved in NADES 10 was capable of penetrating the nail.

### References:

- Baswan, S., Kasting, G.B., Li, S.K., Wickett, R., Adams, B., Eurich, S., Schamper, R., 2017. Understanding the formidable nail barrier: a review of the nail microstructure, composition and diseases. Mycoses 60, 284-295. https://doi.org/10.1016/j.physbeh.2017.03.040
- Brown, M., Turner, R., Wevrett, S.R., 2018. Use of in vitro performance models in the assessment of drug delivery across the human nail for nail disorders. Expert Opin. Drug Deliv. 15, 983-989. https://doi.org/10.1080/17425247.2018.1518425
- Christenson, J.K., Peterson, G.M., Naunton, M., Bushell, M., Kosari, S., Baby, K.E., Thomas, J., 2018. Challenges and opportunities in the management of onychomycosis. J. Fungi 4, 87. https://doi.org/10.3390/jof4030087
- Del Rosso, J.Q., 2014. The role of topical antifungal therapy for onychomycosis and the emergence of newer agents. J. Clin. Aesthet. Dermatol. 7, 10-18.
- Elkeeb, R., Hui, X., Maibach, H.I., 2017. Nail penetration, in: Textbook of Cosmetic Dermatology. CRC Press, pp. 32-41.
- Faergemann, J., Baran, R., 2003. Epidemiology, clinical presentation and diagnosis of onychomycosis. Br. J. Dermatology, Suppl. 149, 1-4. https://doi.org/10.1046/j.1365-2133.149.s65.4.x
- Grzelak EM, Jesionek W, Majer-Dziedzic B, Choma IM. Applications of novel direct bioautography tests for analysis of antimicrobials: a review J AOAC Int. 2013 Nov-Dec;96(6):1167-74
- Gupta, A.K., Stec, N., 2019. Recent advances in therapies for onychomycosis and its management. F1000Research 8, 1-7.
- Lipner, S.R., Scher, R.K., 2019. Onychomycosis: Clinical overview and diagnosis. J. Am. Acad. Dermatol. https://doi.org/10.1016/j.jaad.2018.03.062
- Mustafa NR, Spelbos VS, Witkamp GJ, Verpoorte R, Choi YH. Solubility and Stability of Some Pharmaceuticals in Natural Deep Eutectic Solvents-Based Formulations. Molecules. 2021 Apr 30;26(9):2645. doi: 10.3390/molecules26092645
- Shirwaikar, A.A., Thomas, T., Shirwaikar, A., Lobo, R., Prabhu, K.S., 2008. Treatment of onychomycosis: An update. Indian J. Pharm. Sci. 70, 710-714. https://doi.org/10.4103/0250-474X.49088
- Snyder, 1974. Classification of the solvent properties of common liquids J. Chrom. A 92:223-230
- Westerberg, D.P., Voyack, M.J., 2013. Onychomycosis: Current Trends in Diagnosis and Treatment. Am. Fam. Physician 88, 762-770.

## Claims

1. A plant extract from a food crop residue for use in a method of treatment of a fungal infection, said food crop belonging to the family of Solanaceae.

2. The plant extract for use according claim 1 comprising sebacic acid or a salt thereof.

3. Sebacic acid or a salt thereof for use in a method of treatment of a fungal infection.

4. The plant extract for use according to claim 1 or 2 or sebacic acid or a salt thereof for use according to claim 3, wherein the antifungal infection is an infection caused by the fungus *Trichophyton,* preferably *Trichophyton mentagrophytis* and/or *Trichophyton rubrum.*

5. The plant extract for use according to claim 1, 2 or 4 or sebacic acid or a salt thereof for use according to claim 3 or 4, wherein the fungal infection is onychomycosis.

6. The plant extract for use according to claim 1, 2, 4 or 5 or sebacic acid or a salt thereof for use according to any one of claims 3-5, wherein the method comprises topical administration of said plant extract on or near an infected area, preferably wherein the plant extract is applied on a nail, hair or skin.

7. The plant extract for use according to claims 1, 2, 4-6 or sebacic acid or a salt thereof for use according to any one of claims 2 to 6, wherein said food crop residue is selected from *Solanum tuberosum, Solanum lycopersicum, Solanum melongena* and *Capsicuum annuum.*

8. The plant extract for use according to claim 1, 2, 4-7 or sebacic acid or a salt thereof for use according to any one of claims 2 to 7, wherein said extract comprises at least one compound that is characterized with a retention factor (Rf) value of between about 0.3 and about 0.35 as determinable with thin layer chromatography and using silica gel 60 F254 as stationary phase and a mixture of chloroform, acetone and formic acid in a (v/v) ratio of 75:16.5:8.5 as liquid phase.

9. The plant extract for use according to claim 1, 2, 4-8, wherein the extract is obtainable by a method, comprising the steps of
a) obtaining an aqueous juice from residue of a food crop belonging to the family of Solanaceae;
b) fractionating said aqueous juice with chromatography using a non-polar stationary phase, preferably a stationary phase comprising a copolymer styrene-divinylbenzene adsorbent resin, to obtain a fractionated juice;
c) drying the fractionated juice to obtain a dried fractionated juice;
d) reconstituting the dried fractionated juice in an organic solvent or organic solvent mixture to obtain a crude extract;
e) removing undissolved solids from the crude extract;
f) removing the organic solvent from the crude extract obtained in step e) to obtain a dried crude extract;
g) reconstituting the dried crude extract in water and a non-water-miscible organic solvent or non-water-miscible organic solvent mixture under vigorous agitation;
h) separating the non-water-miscible organic solvent or non-water-miscible organic solvent mixture from the water; and
i) removing the non-water-miscible organic solvent to obtain the plant extract.

10. A composition comprising sebacic acid or a salt thereof, or a plant extract as defined in any one of claims 1, 2, 4-9 and a suitable carrier.

11. The composition according to claim 10, wherein said suitable carrier is a natural deep eutectic solvent (NADES), preferably comprising one or more of 1,2-propanediol, 1,3-butanediol, aconitic acid, adonitol, α-L-rhamnose, β-alanine, betaine, choline chloride, citric acid, D-(-)-fructose, D-(+)-galactose, D-(+)-glucose, D-(+)-trehalose, DL-malic acid, D-mannose, D-proline, D-sorbitol, D-xylose, glycerol, glycol, glycolic acid, glycine, inositol, L-(+)-tartaric acid, lactic acid, 1-glutamic salt, L-proline, L-serine, malic acid, malonic acid, maltose, meso-erythritol, oxalic acid, phytic acid sodium, proline, propylene glycol, quercetin, raffinose, ribitol, sorbose, sucrose, tri-ethylene glycol, urea, xylitol, more preferably urea and/or lactic acid.

12. The composition according to claim 11, wherein the NADES is selected from mixtures of (i) urea, glucose and citric acid; (ii) lactic acid and sodium acetate; (iii) lactic acid, glycine and water; (iv) lactic acid and beta-alanine; (v) glycolic acid and betaine; (vi) urea, lactic acid, propylene glycol and glycerol; (vii) urea, lactic acid and propylene glycol; (viii) urea and proline; (ix) urea and glycerin; (x) urea and betaine.

13. The composition according to any one of claims 10 to 12 for use in a method of treatment of a fungal infection, preferably, wherein the antifungal infection is an infection caused by the fungus *Trichophyton,* more preferably *Trichophyton mentagrophytis* and/or *Trichophyton rubrum.*

14. Use of a plant extract as defined in any one of claims 1-9, sebacic acid or a salt thereof, or a composition according to any one of claims 10-12 as a disinfectant.

15. An object treated with an extract as defined in any one of claims 1-9, sebacic acid or a salt thereof, or a composition according to any one of claims 10-12, preferably wherein the object is a shoe, a sock, clothing or a surface.
